(19) Europäisches Patentamt
European Patent Office
Office européen des brevets

(11) Veröffentlichungsnummer : **0 144 690**
**B1**

(12) # EUROPÄISCHE PATENTSCHRIFT

(45) Veröffentlichungstag der Patentschrift : **20.01.88**

(51) Int. Cl.⁴ : **C 07 D487/08**

(21) Anmeldenummer : **84112930.7**

(22) Anmeldetag : **26.10.84**

(54) **Substituierte Triazanonane.**

(30) Priorität : **05.11.83 DE 3340077**

(43) Veröffentlichungstag der Anmeldung : **19.06.85 Patentblatt 85/25**

(45) Bekanntmachung des Hinweises auf die Patenterteilung : **20.01.88 Patentblatt 88/03**

(84) Benannte Vertragsstaaten : **CH DE GB LI**

(56) Entgegenhaltungen :
**JOURNAL OF THE CHEMICAL SOCIETY, 1968, Sektion C, Teil II A. ALBERT, H. YAMAMOTO "Quinazoline Studies. Part XII" Seiten 1944-1949**
**INORGANIC CHEMISTRY, Vol. 8, 1969 L.T. TAYLOR, D.H. BUSCH "Stereo-specific Formation of Chelate Sandwich Compounds Derived from o-Aminobenzaldehyde and 2-Amino-5-chlorobenzaldehyde by a Metal Ion Induced Rearrangement" Seiten 1366-1371**
**TETRAHEDRON, Vol. 36, No. 13, 1980 P. CALUWE "Heteroannelations with o-Aminoaldehydes" Seiten 2359-2407**
**AUSTRALIAN JOURNAL OF CHEMISTRY, Vol. 23, No. 9, 1970 D. ST. C. BLACK, M.J. LANE "Metal Template Reactions" Seiten 2055-2065**
**JOURNAL OF THE CHEMICAL SOCIETY, Perkin Transactions II, 1979 A.R. KATRITZKY, V.J. BAKER, I.J. FERGUSON, R.C. PATEL "The Conformational Analysis of Saturated Heterocycles. Part 87" Seiten 143-150**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17e**
**D-6710 Frankenthal (DE)**
Erfinder : **Kohler, Rolf-Dieter, Dr.**
**Amselweg 3**
**D-6803 Edingen-Neckarhausen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Verfahren zur Herstellung von Verbindungen der allgemeinen Formel I

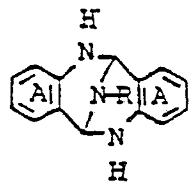

in der R Wasserstoff, gegebenenfalls durch Hydroxy, Alkoxy, Cyan, Amino, Alkylamino, Dialkylamino, Alkylmercapto oder Aryl substituiertes $C_1$- bis $C_{12}$-Alkyl, $C_3$- bis $C_{10}$-Alkenyl oder gegebenenfalls durch Hydroxy substituiertes Cycloalkyl mit 5 bis 7 Ringgliedern ist und die Ringe A noch durch Fluor, Chlor oder Brom substituiert sein und/oder einen annellierten Ring tragen können. Bei dem Verfahren setzt man Verbindungen der Formel II

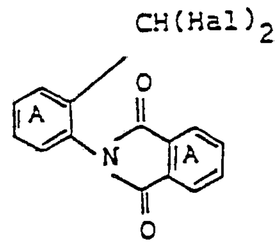

mit Aminen der Formel $RNH_2$ gegebenenfalls in Gegenwart von Lösungsmitteln um ; Hal ist dabei Chlor oder Brom.

Einzelne Alkylreste R sind beispielsweise : Hydroxyethyl, Hydroxypropyl, Methoxyethyl, Ethoxyethyl, Butoxyethyl, Methoxypropyl, Cyanoethyl, Aminoethyl, Dimethylaminoethyl oder Methylmercaptoethyl sowie

$$CH_2-CH=CH-CH_2OH, \quad CH_2-\underset{CH_3}{C}=CH-CH_2OH$$

Hydroxycyclopentyl oder Hydroxycyclohexyl.

Als Lösungsmittel sind z. B. Chlorkohlenwasserstoffe, Alkohole, Glykole, Glykolether, Amide, Ether oder Ketone geeignet.

Einzelne Lösungsmittel sind beispielsweise : Methylenchlorid, Chloroform, Methanol, Ethanol, Propanol, Isopropanol, Ethylenglykol, Methylglykol, Formamid, Dimethylformamid, Ethylenglykoldimethylether oder Aceton.

Einzelheiten der Reaktionsführung können den Beispielen entnommen werden, in denen sich Angaben über Teile und Prozente, sofern nicht anders vermerkt, auf das Gewicht beziehen.

Die Verbindungen der Formel II kann man durch Halogenierung der Verbindungen der Formel

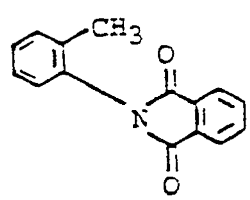

nach an sich bekannten Methoden herstellen.

Die Verbindungen der Formel I sind verkappte 2-Aminobenzaldehyde, die durch Säuren oder Basen in Freiheit gesetzt und so für weitere Umsetzungen bereitgestellt werden können. Die Verbindungen der Formel I können somit als eine stabile Form der an sich in der Regel instabilen 2-Aminobenzaldehyde betrachtet werden.

Aus dem Journal of the Chemical Society 1968, Section C, Teil II, S. 1947 sind schon Verbindungen vom Typ der Formel I bekannt, diese wurden jedoch ausgehend von den bisher kaum zugänglichen o-Aminobenzaldehyden gewonnen.

2-Aminobenzaldehyde sind wertvolle Zwischenprodukte zur Herstellung von Farbstoffvorprodukten,

Wirkstoffen und Heterocyclen (s. z. B. Tetrahydron 36, 2359 (1980)).

Von besonderer Bedeutung sind Verbindungen der Formel I, bei denen R Hydroxyethyl ist und die Ringe A unsubstituiert sind oder noch durch Chlor substituiert sein können.

## Beispiel 1

N-(Hydroxyethyl)-dibenzo [c,g]-2,6,9-triazabicyclo-[3,3,1] nonan

612 g N-(2-Dichlormethyl-phenyl)-phthalimid wurden portionsweise unter Rühren in 1 200 g Ethanolamin eingetragen. Die Temperatur stieg dabei auf 80 °C an. Nach 2 Stunden wurden 3 000 g Eiswasser zugegeben, der Niederschlag wurde abgesaugt und mit Wasser neutral gewaschen. Nach Trocknen im Vakuum wurden 255 g (96 % d. Th.) farbloses Produkt vom Fp. 196 °C erhalten.

## Beispiel 2

N-(Hydroxyethyl)-bis-(2-chlorbenzo)-[c,g]-2,6,9-triazabicyclo-[3,3,1] nonan

Unter Rühren wurden bei 70 °C 68 g N-(3-Chlor-2-dichlormethyl-phenyl)-phthalimid zu 150 g Ethanolamin gegeben. Nach 1 Stunde wurde auf 20 °C gekühlt und anschließend wurden 400 g Eiswasser zugetropft. Der Niederschlag wurde abgesaugt, mit Wasser gewaschen und im Vakuum getrocknet. Ausbeute 28 g (84 % d. Th.), Fp. 176 °C.

## Beispiel 3

N-(Aminoethyl)-dibenzo [c,g]-2,6,9-triazabicyclo-[3,3,1] nonan

Zu 40 g 1,2-Diaminoethan wurden unter Rühren bei 40 °C portionsweise 20 g N-(2-Dichlormethyl-phenyl)-phthalimid gegeben. Nach 2 Stunden wurden bei 20 °C 150 g Eiswasser zugetropft, der Niederschlag wurde abgesaugt und mit Wasser neutral gewaschen. Nach dem Trocknen im Vakuum wurden 8 g (88 % d. Th.) farbloses Produkt erhalten, das bei 182 °C schmolz.

Entsprechend Beispiel 1 können Verbindungen der Formel

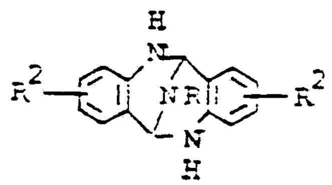

hergestellt werden.

| Bsp. | R | $R^2$ |
|---|---|---|
| 4 | $CH_2CH_2OH$ | 3-Cl |
| 5 | $CH_2CH_2OH$ | 4-Cl |
| 6 | $CH_2CH_2OH$ | 5-Cl |
| 7 | $CH_2CH_2OH$ | 2-Br |
| 8 | $CH_2CH_2OH$ | 3-Br |
| 9 | $CH_2CH_2OH$ | 4-Br |
| 10 | $CH_2CH_2OH$ | 5-Br |
| 11 | $CH_2CH_2NH_2$ | 2-Cl |
| 12 | $CH_2CH_2N(CH_3)_2$ | H |
| 13 | $CH_2CH_2N(CH_3)_2$ | 2-Cl |
| 14 | $CH_2-\underset{OH}{CH}-CH_3$ | H |
| 15 | $CH_2-\underset{OH}{CH}-CH_3$ | 2-Cl |
| 16 | $CH_2-\underset{OH}{C}-CH_3$ | H |

**Patentanspruch**

Verfahren zur Herstellung von Verbindungen der allgemeinen Formel I

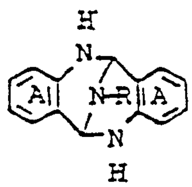

in der R Wasserstoff gegebenenfalls durch Hydroxy, Alkoxy, Cyan, Amino, Alkylamino, Dialkylamino, Alkylmercapto oder Aryl substituiertes $C_1$- bis $C_{12}$-Alkyl, $C_3$- bis $C_{10}$-Alkenyl oder gegebenenfalls durch Hydroxy substituiertes Cycloalkyl mit 5 bis 7 Ringgliedern ist und die Ringe A noch durch Fluor, Chlor oder Brom substituiert sein und/oder einen annellierten Ring tragen können, dadurch gekennzeichnet, daß man Verbindungen der Formel II

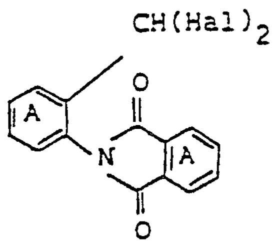

mit Aminen der Formel $RNH_2$ gegebenenfalls in Gegenwart von Lösungsmitteln umsetzt, Hal ist dabei Chlor oder Brom.

**Claim**

A process for the preparation of a compound of the formula I

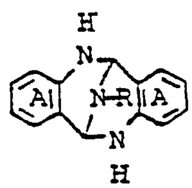

where R is hydrogen, $C_1$-$C_{12}$-alkyl which is unsubstituted or substituted by hydroxyl, alkoxy, cyano, amino, alkylamino, dialkylamino, alkylmercapto or aryl, $C_3$-$C_{10}$-alkenyl, or unsubstituted or hydroxyl-substituted cycloalkyl having from 5 to 7 ring members and the rings A may further-more be substituted by fluorine, chlorine or bromine and/or may carry a fused ring, wherein a compound of the formula II

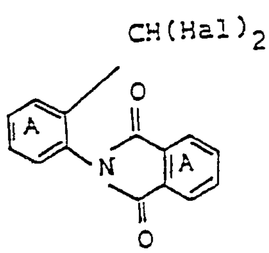

where Hal is chlorine or bromine, is reacted with an amine of the formula $RNH_2$ in the presence or absence of a solvent.

**Revendication**

Procédé de préparation de composés de la formule générale I

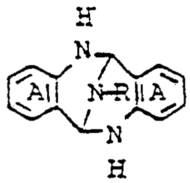

dans laquelle R désigne un atome d'hydrogène, un radical alkyle en $C_1$ à $C_{12}$ éventuellement hydroxy-, alcoxy-, cyano-, amino-, alkylamino-, dialkylamino-, alkylmercapto- ou aryl- substitué, un groupe alcényle en $C_3$ à $C_{10}$ ou un groupe cycloalkyle avec cinq à sept chaînons dans le cycle, éventuellement hydroxy-substitué, et les noyaux A peuvent en outre être substitués par du fluor, du chlore ou du brome et(ou) porter un autre cycle condensé, caractérisé en ce que l'on fait réagir des composés de la formule II

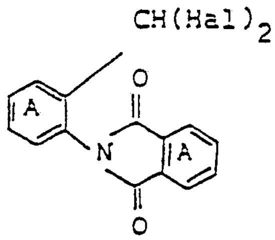

dans laquelle Hal désigne le chlore ou le brome, avec des amines de la formule $RNH_2$, éventuellement en présence d'un solvant.